# EUROPEAN PATENT APPLICATION

(11) **EP 4 834 232 A1**
(43) Date of publication of application: **04.11.2026**
(21) Application number: 26175799.1
(22) Date of filing: 30.04.2026
(51) Int. Cl.: A61B 6/03, A61B 6/00

(54) **SYSTEM AND METHOD FOR ROTATION ANGLE FEEDBACK, AND READABLE STORAGE MEDIUM**

(30) Priority: 30.04.2025 CN 202510577431
(71) Applicant: Shanghai United Imaging Healthcare Co., Ltd., Shanghai 201807 (CN)
(72) Inventor: TIAN, Xi, Shanghai, 201807 (CN)
(74) Representative: Murgitroyd & Company

(57) **Abstract**

A system and a method for rotation angle feedback, and a readable storage medium applied to a CT device are provided. The system includes a detection module and a processing module. The CT device has a gantry including a stator and a rotor, and the gantry is provided with the detection module and a plurality of indexing marks arranged along a circumference centered on a scanning center of the CT device. The detection module detects the indexing marks during rotation of the rotor to generate a pulse signal and transmits the pulse signal to the processing module. The processing module is configured to, in response to receiving the pulse signal corresponding to one of the indexing marks, determine a target rotation angle corresponding to an acquired image based on a present angular speed and a rotation duration from the one of the indexing marks.

## Description

### TECHNICAL FIELD

The present disclosure relates to the field of medical device technologies, and in particular, to a system and a method for rotation angle feedback, and a readable storage medium.

### BACKGROUND

In computed tomography (CT) imaging, three-dimensional CT images can be constructed by combining and processing two-dimensional images acquired at different rotation angles. Accurate rotation angles serve as a basis for reconstruction algorithms. Inaccurate rotation angles cause distortion and artifacts in CT images.

In related technologies, various types of rotation angle encoders based on different principles are employed to acquire angle information regarding a CT tube. These rotation angle encoders feedback the detected angle information to a controller of the CT device, and the controller performs image reconstruction based on the angle information. Examples of such encoders include magnetic scales with read heads, steel scales with read heads, grating scales with read heads, and rotary encoders.

However, conventional rotation angle encoders for rotation angle information feedback suffer from high costs.

### SUMMARY

According to various embodiments, a system and a method for rotation angle feedback, and a readable storage medium are provided.

In a first aspect, a system for rotation angle feedback applied to a CT device is provided in the present disclosure. The system includes a detection module and a processing module. The CT device has a gantry including a stator and a rotor, and the gantry is provided with the detection module and a plurality of indexing marks arranged along a circumference centered on a scanning center of the CT device. The detection module is configured to detect the indexing marks during rotation of the rotor to generate a pulse signal, and transmit the pulse signal to the processing module. The processing module is configured to, in response to receiving the pulse signal corresponding to one of the indexing marks, determine a target rotation angle corresponding to an acquired image based on a present angular speed and a rotation duration from the one of the indexing marks.

In some embodiments, the processing module is further configured to generate counting pulses corresponding to the present angular speed , and determine the target rotation angle corresponding to each acquired image during the rotation of the rotor based on the counting pulses between the indexing marks.

In some embodiments, the processing module is further configured to determine a count value corresponding to each acquired image based on the counting pulses, and determine the target rotation angle corresponding to each acquired image during the rotation of the rotor based on each count value and a counting duration required for each count value.

In some embodiments, the processing module includes an acquisition module and a synchronization module. The acquisition module is connected to the detection module and a slip ring of the CT device, respectively. The synchronization module is connected to the slip ring and a driving module of the CT device.

In some embodiments, the count value is used to instruct the driving module to control the angular speed of the rotor based on the count value and a theoretical count value.

In some embodiments, the acquisition module is configured to transmit the pulse signal and the counting pulses to the synchronization module through the slip ring. The synchronization module is configured to transmit the pulse signal to the driving module.

In some embodiments, the acquisition module is configured to determine, based on a first ratio of a duration required for one revolution of the rotor to a number of counting pulses per revolution of the rotor, a counting duration required for generating each count value, and determine, based on a second ratio of the counting duration required for generating each count value and a minimum clock cycle for the acquisition module, a number of minimum clock cycles required for generating the count value.

In some embodiments, the acquisition module is further configured to use the second ratio as the number of minimum clock cycles required for generating the count value on a condition that the second ratio is an integer, and compensate the second ratio based on a remainder in the second ratio to obtain the number of minimum clock cycles required for generating the count value on a condition that the second ratio is a non-integer.

In some embodiments, the plurality of indexing marks are disposed on one of the stator and the rotor, and the detection module is disposed on the other of the stator and the rotor.

In some embodiments, the plurality of indexing marks include a plurality of blind holes arranged at equal angular intervals around the scanning center.

In some embodiments, the plurality of blind holes include one zero-position hole and a plurality of positioning holes. The detection module includes a first detection module configured to detect the zero-position hole and a second detection module configured to detect the plurality of positioning holes.

In some embodiments, the synchronization module is further configured to feed back the target rotation angle to a controller of the CT device.

In some embodiments, the at least two of the indexing marks include two adjacent indexing marks, and the acquisition module is configured to generate the counting pulses between rising edges or falling edges of pulse signals corresponding to the two adjacent indexing marks.

In some embodiments, the synchronization module is further configured to determine an actual rotation duration between the adjacent indexing marks based on the count value, and transmit the actual rotation duration to the driving module. The actual rotation duration is used to instruct the driving module to control the angular speed of the rotor based on a difference between the actual rotation duration and a theoretical rotation duration between the adjacent indexing marks.

In a second aspect, a method for rotation angle feedback applied to a system for rotation angle feedback is provided in the present disclosure. The system includes a processing module and a detection module. A plurality of indexing marks arranged along a circumference centered on a scanning center of a CT device and the detection module are disposed on a gantry of the CT device. The gantry includes a stator and a rotor. The method includes: in response to receiving the pulse signal corresponding to one of the indexing marks, determine a target rotation angle corresponding to an acquired image based on a present angular speed and a rotation duration from the one of the indexing marks, the pulse signal being generated and transmitted by the detection module in response to detecting the indexing marks during rotation of the rotor.

In a third aspect, an electronic device including a memory and a processor is further provided in the present disclosure. The memory stores a computer program. The computer program, when executed by a processor, causes the processor to implement the steps of the method according to the second aspect.

In a fourth aspect, a non-transitory computer-readable storage medium on which a computer program is stored is further provided in the present disclosure. The computer program, when executed by a processor, causes the processor to implement the steps of the method according to the second aspect.

In a fifth aspect, a computer program product including a computer program is further provided in the present disclosure. The computer program, when executed by a processor, causes the processor to implement the steps of the method according to the second aspect.

In a sixth aspect, a CT device is further provided in the present disclosure. The CT device includes a gantry, a plurality of indexing marks, a sensor, and a controller. The gantry includes a stator and a rotor. The plurality of indexing marks are provided on one of the stator and the rotor and are arranged at angular intervals along a circumference centered on a scanning center of the CT device. The sensor is provided on the other of the stator and the rotor and is configured to detect the indexing marks during rotation of the rotor to generate a pulse signal. The controller is configured to, in response to receiving the pulse signal corresponding to one of the indexing marks, determine a target rotation angle corresponding to an acquired image based on a present angular speed and a rotation duration from the one of the indexing marks.

### BRIEF DESCRIPTION OF THE DRAWINGS

Features and advantages of the present disclosure will be better understood from the following detailed description of exemplary embodiments employing the principles of the present disclosure with reference to the following drawings:
FIG. 1 is a schematic diagram of an angle feedback system based on a rotation angle encoder according to an embodiment;
FIG. 2 is a schematic diagram of a pulse signal according to an embodiment;
FIG. 3 is a schematic diagram of a system for rotation angle feedback according to an embodiment;
FIG. 4 is a schematic diagram of a system for rotation angle feedback according to another embodiment;
FIG. 5 is a schematic diagram of a system for rotation angle feedback according to another embodiment;
FIG. 6 is a schematic diagram of a system for rotation angle feedback according to another embodiment; and
FIG. 7 is a flow diagram illustrating a method for rotation angle feedback according to an embodiment.

### DETAILED DESCRIPTION OF THE EMBODIMENTS

To make the objectives, technical solutions, and advantages of the present disclosure clearer, the present disclosure is described in further detail below with reference to the accompanying drawings and embodiments. It should be understood that the specific embodiments described herein are merely used to explain the present disclosure and are not intended to limit the present disclosure.

In the description of the present disclosure, it should be understood that the orientations or positional relationships indicated by the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential" and the like are based on the orientations or positional relationships shown in the accompanying drawings. These terms are merely for the convenience of describing the present disclosure and simplifying the description, rather than indicating or implying that the referred device or element must have a specific orientation, or be constructed and operated in a specific orientation, and therefore cannot be interpreted as limiting the present disclosure.

In the present disclosure, the terms "first", "second" and the like are used for descriptive purposes only and cannot be understood as indicating or implying relative importance or implicitly indicating the number of indicated technical features. Thus, a feature defined with "first" or "second" may explicitly or implicitly include at least one of such features. In the description of the present disclosure, "a plurality of" means at least two, for example, two, three, etc., unless otherwise specifically defined.

In the present disclosure, unless otherwise expressly specified and defined, the terms "mounted", "connected", "coupled", "fixed" and the like should be understood in a broad sense. For example, they may refer to a fixed connection, a detachable connection, or an integral connection, they may refer to a mechanical connection or an electrical connection, they may refer to a direct connection or an indirect connection via an intermediate medium, and they may refer to internal communication between two elements or interaction between two elements, unless otherwise expressly specified and limited. Those of ordinary skill in the art can understand the specific meanings of the above terms in the present disclosure according to specific situations.

In the present disclosure, unless otherwise expressly specified and defined, a first feature being "on" or "under" a second feature may mean that the first and second features are in direct contact, or that the first and second features are in indirect contact via an intermediate medium. Furthermore, the first feature being "above", "over", or "on top of" the second feature may mean that the first feature is directly above or obliquely above the second feature, or simply that the horizontal height of the first feature is higher than that of the second feature. The first feature being "below", "under", or "underneath" the second feature may mean that the first feature is directly below or obliquely below the second feature, or simply that the horizontal height of the first feature is lower than that of the second feature.

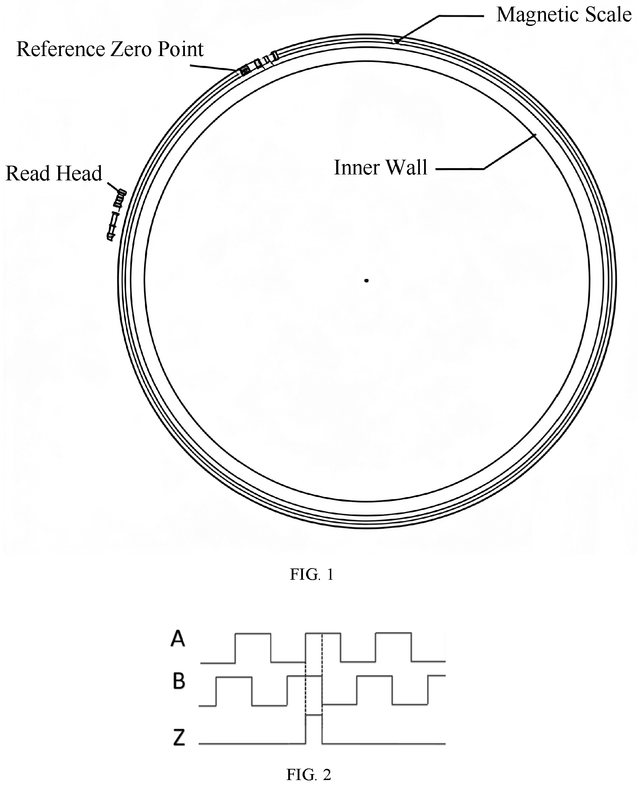

As shown in FIG. 1, various types of rotation angle encoders based on different principles (such as magnetic scales with read heads, steel scales with read heads, grating scales with read heads, and rotary encoder) are presently used to acquire rotation angles of a CT tube. The principle involves relative motion between the read head and the magnetic scale, steel scale, or grating scale. For example, the read head reads magnetic pole signals of the magnetic scale to generate A/B pulse signals as shown in FIG. 2, and further generates a Z pulse signal representing a zero position when the read head passes the zero position of the magnetic scale. The A/B pulse signals provided by the read head are counted using quadrature decoding. For example, 1 and 0 represent high and low levels of the A/B signals, respectively, where "10" represents that the A signal is at a high level and the B signal is at a low level. When a combination of high and low levels of the A/B signals sequentially changes from "10" to "00", from "00" to "01", from "01" to "11", and from "11" to "10", the count is incremented by 1 for each change. When the combination sequentially changes from "10" to "11", from "11" to "01", from "01" to "00", and from "00" to "10", the count is decremented by 1 for each change. When the combination changes from "10" to "01", from "01" to "10", from "00" to "11", or from "11" to "00", the count remains unchanged.

In actual use, as the read head and the magnetic scale move relative to each other to generate a raw pulse signal, an acquisition circuit reads the raw pulse signal using the above quadrature decoding method and converts it into a count value. The count value is then used to control the number of samples per revolution. To ensure measurement accuracy, the number of raw pulses provided by the magnetic scale should be at least about a dozen times the number of samples per revolution. Assuming a total grid length of the magnetic scale is 3.0 m with a resolution of 0.005 mm, the total number of raw pulses per revolution of the CT device is 600000 (3000 mm / 0.005 mm = 600000). A larger value indicates higher accuracy of the magnetic scale and higher cost. The pulse count corresponding to the linear length of the magnetic scale is converted into a rotation angle value per revolution to provide feedback information corresponding to a rotation angle for each image of the CT device.

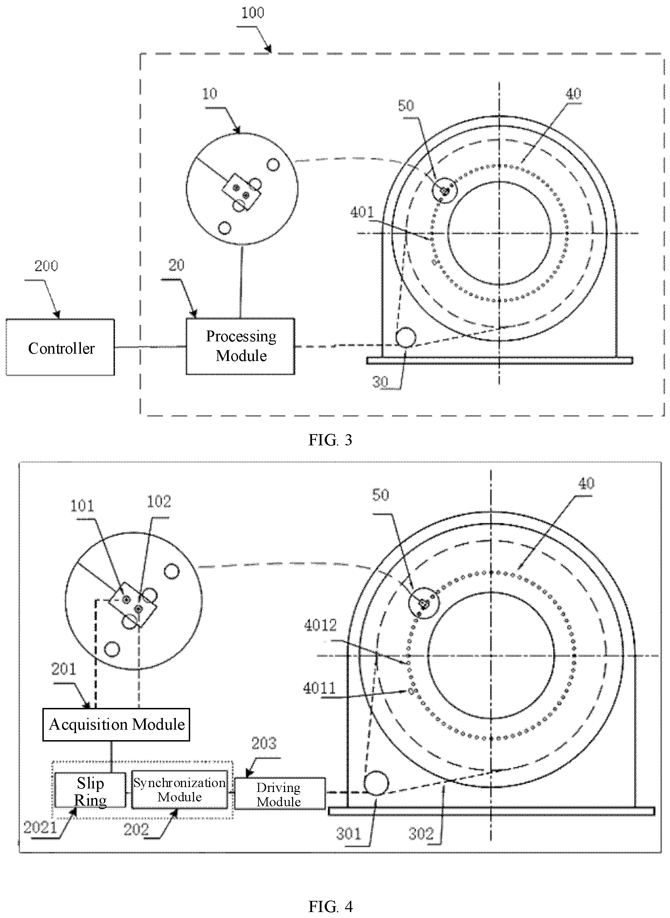

The system for rotation angle feedback in some embodiments is applied to a CT device. The CT device includes a gantry, which includes a stator and a rotor, the rotor being rotatable relative to the stator. The gantry is further provided with a power device for driving the rotor to rotate, a detector for acquiring images, an X-ray generator, and other components. FIG. 3 is a schematic diagram of a system for rotation angle feedback according to an embodiment, applied to a CT device. As shown in FIG. 3, the system 100 for rotation angle feedback includes a detection module 10 and a processing module 20. A gantry of the CT device includes a stator 40 and a rotor 50. A plurality of indexing marks arranged along a circumference centered on a scanning center of the CT device and the detection module 10 are disposed on the stator 40 and the rotor 50 of the gantry, respectively. The detection module 10 is configured to detect the indexing marks during rotation of the rotor 50 to generate a pulse signal, and transmit the pulse signal to the processing module 20. The processing module 20 is configured to, in response to receiving the pulse signal corresponding to one of the indexing marks, determine a target rotation angle corresponding to an acquired image based on a present angular speed and a rotation duration from the one of the indexing marks.

In some embodiments, when the detection module 10 on the rotor 50 rotates to detect any one of the indexing marks, the detection module 10 generates a corresponding pulse signal and transmits the pulse signal to the processing module 20. Upon receiving the pulse signal corresponding to one of the indexing marks, the processing module 20 determines a target rotation angle corresponding to an acquired image based on a present angular speed and a rotation duration from the one of the indexing marks. When the sensor serving as the detection module 10 on the rotor side of the CT device rotates and detects a hole signal of the indexing marks arranged in a circle on the stator 40, the processing module 20 obtains angle information of a position reached by the rotor 50. For real-time angle feedback between adjacent indexing marks, the processing module 20 calculates an angle traveled by the rotor 50 through integration of the angular speed of the rotor and the rotation duration starting from the one of the indexing marks, so as to obtain real-time rotation angle feedback information of the rotor 50 during continuous rotation.

In some embodiments, the processing module 20 is configured to generate counting pulses corresponding to a present angular speed, and determine a target rotation angle corresponding to each acquired image during the rotation of the rotor 50 based on the counting pulses between the indexing marks. The acquired image refers to a projection image acquired by the CT device during rotational scanning, and the target rotation angle is a projection angle corresponding to the projection image. The counting pulses between the indexing marks refers to the counting pulses occurring between two indexing mark pulses.

In the present disclosure, the counting pulses generated by the processing module are not limited to a specific type. In an exemplary embodiment, the counting pulses may be isochronous counting pulses.

The detection module 10 and the plurality of indexing marks arranged along a circumference centered on the scanning center of the CT device are disposed on the gantry. The detection module 10 is a hardware detection unit, which can include a proximity switch. The proximity switch is configured to detect the indexing marks on the stator 40 without contact during rotation of the rotor 50, so as to generate 24V DC high-level and low-level pulse signals, and output the pulse signals to the acquisition module 201. The indexing marks may be physical marks uniformly distributed along a circle on the stator or the rotor for angular positioning, and can be recognized by the detection module 10. Optionally, the plurality of indexing marks may be disposed on the stator 40 (e.g., on an inner side of a stator fixing plate) with the detection module 10 disposed on the rotor 50, or alternatively, the plurality of indexing marks may be disposed on the rotor 50 with the detection module 10 disposed on the stator 40.

The pulse signals output by the detection module 10 when detecting at least two of the indexing marks may correspond to indexing marks adjacent in a circumferential direction or spaced apart in the circumferential direction.

The plurality of indexing marks may take various forms, including, but not limited to, a plurality of blind holes 401 arranged at equal angular intervals centered on the scanning center, or alternatively, raised structures, magnetic marks, and optical marks.

In some embodiments, the plurality of indexing marks include a plurality of blind holes 401 arranged at equal angular intervals centered on the scanning center. As shown in FIG. 3, taking the arrangement where the plurality of blind holes 401 are disposed on the stator 40 and the detection module 10 is disposed on the rotor 50 as an example, a circle of sparse blind holes 401 are uniformly arranged on the stator 40 centered on the scanning center. A position of each blind hole 401 corresponds to a fixed angle within a 360° rotation range of the tube on the rotor 50. Specifically, based on the number of blind holes 401, angle information of one revolution of the CT device can be divided into a plurality of arc-shaped sector angles, where each sector angle = 360°/n, and n refers to a number of blind holes 401.

As shown in FIG. 4, the plurality of blind holes 401 include one zero-position hole 4011 and a plurality of positioning holes 4012. Specifically, any one of the plurality of blind holes 401 is designated as the zero-position hole 4011 for determining a rotation start reference (0° position) of the rotor. The remaining blind holes 401 other than the zero-position hole 4011 serve as positioning holes as indexing references for angle calculation. The detection module 10 includes a first detection module 101 configured to detect the zero-position hole 4011 and a second detection module 102 configured to detect the plurality of positioning holes 4012. The detection module 10 is mounted on the rotor 50 via an adjustable fixing bracket, and a probe of the detection module 10 is aligned with a center of each blind hole 401. On a condition that the detection module 10 rotates to detect a blind hole 401, a pulse signal is generated, so that angle information of the rotor 50 can be obtained based on the pulse signal. The first detection module 101 is configured to detect the zero-position hole 4011 to generate a zero-position pulse signal, and the second detection module 102 is configured to detect the positioning holes 4012 to generate a positioning pulse signal. For example, when the second detection module 102 approaches an edge of a positioning hole 4012, an output pulse signal of the second detection module 102 changes from a high level to a low level to generate a falling edge. When the second detection module 102 leaves the positioning hole 4012, the output signal changes from a low level to a high level to generate a rising edge. A rotation angle of the positioning hole 4012 is obtained via the rising edge or falling edge pulse signal output by the second detection module 102.

In an embodiment of the present disclosure, as shown in FIG. 4, the processing module 20 may include an acquisition module 201 and a synchronization module 202.

The acquisition module 201 in the processing module 20 is configured to generate counting pulses corresponding to a present angular speed. Taking two adjacent blind holes 401 as an example, with each blind hole 401 as a timing start point after the CT device rotates to a constant speed, the acquisition module 201 may start timing at a rising edge of a pulse signal output by the detection module 10 when leaving a first blind hole 401, and stop timing at a rising edge of a pulse signal output by the detection module 10 when leaving a second blind hole 401, during which counting pulses corresponding to the present angular speed are generated. Alternatively, the acquisition module 201 may start timing at a falling edge of a pulse signal output by the detection module 10 when approaching the first blind hole 401, and stop timing at a falling edge of a pulse signal output by the detection module 10 when approaching the second blind hole 401, during which counting pulses corresponding to the present angular speed are generated. The counting pulses refer to counting pulses generated at fixed and equal time intervals, which are used to represent a change in a rotation angle of the rotor between two indexing marks.

The processing module 20 is configured to determine a present angular speed, and generate counting pulses during a present adjacent indexing mark interval based on the present angular speed. Specifically, the present angular speed may be calculated by the acquisition module 201 or the synchronization module 202. The previous adjacent indexing mark interval may refer to a completed adjacent indexing mark interval prior to the present adjacent indexing mark interval. The present adjacent indexing mark interval may refer to an adjacent indexing mark interval presently in progress.

In some examples, taking the present angular speed calculated by the acquisition module 201 as an example, the acquisition module 201 may be disposed on a rotor side and configured to latch a rising edge or a falling edge of a pulse signal. When the rotor rotates and the detection module 10 passes a first indexing mark, the detection module 10 detects the indexing mark and outputs a pulse signal corresponding to the indexing mark, and the acquisition module 201 starts timing at an edge of the pulse signal corresponding to the indexing mark. On a condition that the detection module 10 rotates to a next adjacent indexing mark, a pulse signal corresponding to the indexing mark is output, and the acquisition module 201 stops timing at an edge of the pulse signal corresponding to the indexing mark, so as to obtain an actual time interval between pulse signals corresponding to the two indexing marks. The acquisition module 201 calculates a real angular speed corresponding a present indexing mark interval based on a fixed angle between the two indexing marks and the actual time interval. When the rotor enters a rotation interval between a next group of adjacent indexing marks, the acquisition module 201 may use the calculated real angular speed as a present angular speed to generate counting pulses matching the present angular speed.

The count value may be a number of equal-time interval counting pulses generated based on a minimum clock cycle of the acquisition module 201, and is used to determine a rotation angle corresponding to each acquired image during rotation of the CT rotor. Each count value corresponds to a fixed integration time interval, i.e., a counting duration required for each count value. A corresponding rotation angle is obtained by integrating an angular speed with the integration time interval. The acquisition module 201 or the synchronization module 202 may determine a count value corresponding to each acquired image based on the counting pulses, i.e., the count value is a number of equal-time counting pulses to be accumulated between two adjacent fields of view (from an acquired image to a next acquired image). Assuming that 2400 images are acquired per revolution of the rotor 50 and the number of blind holes 401 is 80, 30 images need to be acquired between two blind holes 401. On a condition that a number of isochronous counting pulses per revolution of the rotor 50 is 57600, a number of isochronous counting pulses between two adjacent blind holes 401 is 57600/80 = 720, so a count value between two images is 720/30 = 24.

A counting duration required for generating each count value is determined based on a first ratio of a duration required for one revolution of the rotor 50 to a number of isochronous counting pulses per revolution of the rotor 50 for the acquisition module 201. For example, assuming that the duration required for the rotor 50 to complete one revolution is 499968000 ns, the counting duration required for generating each count value is 499968000/57600 = 8680.

A target rotation angle corresponding to each acquired image during the rotation of the rotor 50 is determined based on a product of the count value and the counting duration required for each count value. The synchronization module 202 may feed back the target rotation angle to a controller 200 of the CT device. The controller 200 may control the CT device to acquire an image corresponding to the target rotation angle based on the received target rotation angle.

In a possible implementation, the detection module 10 is configured to detect the blind hole 401 during rotation of the rotor 50 to generate a pulse signal, and transmit the pulse signal to the acquisition module 201. The acquisition module 201 synchronously transmits the pulse signal to the driving module 203 via the synchronization module 202. The driving module 203 controls an angular speed of the rotor 50 based on the isochronous counting pulses output during the interval between two adjacent blind holes 401.

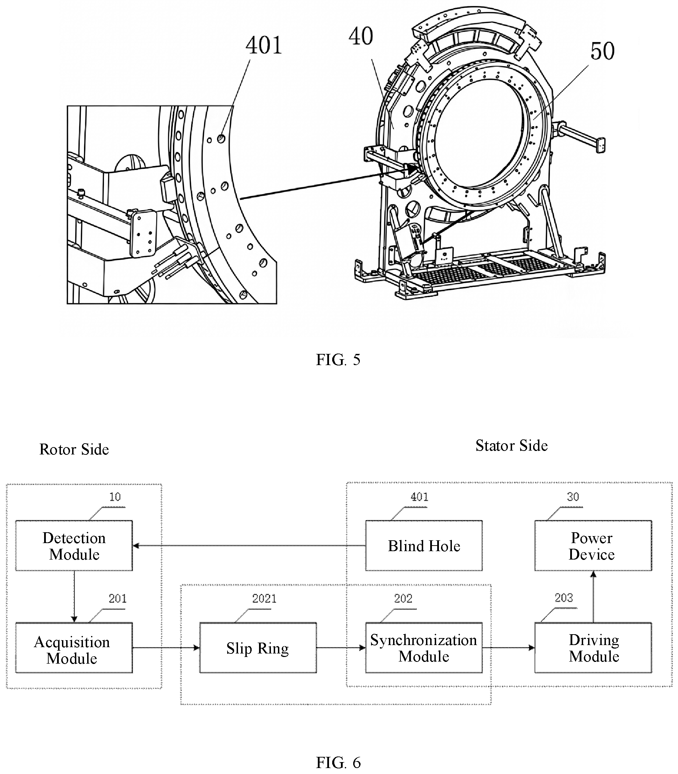

Optionally, as shown in FIG. 5, in an embodiment of the present disclosure, the blind holes 401 may also be uniformly disposed on the rotor 50, and the detection module may also be disposed on the stator 40.

Further, to verify accuracy of target rotation angle feedback of the system for rotation angle feedback provided by the present disclosure, verification is performed on the system for rotation angle feedback before application.

A duration t required for the rotor 50 of the CT device to complete one revolution is divided into n parts. Assuming that a preset angular speed ω₀ (unit: °/s) is issued to a power device 30 driving the rotor 50, and speed fluctuations of the rotor 50 at n time points are Δω₁, Δω₂, Δω₃, Δω₄, ..., and Δωₙ respectively, and a speed at each time point is ωᵢ = ω₀ + Δωᵢ, where i = 1, 2, 3, ..., and n, and n is a positive integer representing the number of time portions into which the duration t for one full rotation of the rotor is equally divided. An actual rotation angle θ(t) under such speed fluctuation is: $\begin{matrix}θ \left(t\right) = \left({ω}_{0}+{Δω}_{1}\right) ∗ \frac{t}{n} + \left({ω}_{0}+{Δω}_{2}\right) ∗ \frac{t}{n} + \left({ω}_{0}+{Δω}_{3}\right) ∗ \frac{t}{n} + \left(\begin{matrix}{ω}_{0} \\ + {Δω}_{4}\end{matrix}\right) ∗ \frac{t}{n} + ⋯ + \left({ω}_{0}+{Δω}_{n}\right) ∗ \frac{t}{n} = \left({nω}_{o}+{\sum }_{i = 1}^{n} {Δω}_{i}\right) ∗ \frac{t}{n} \\ = {ω}_{0} ∗ t + \frac{{\sum }_{i = 1}^{n} {Δω}_{i}}{n} ∗ t\end{matrix}$

An average speed deviation Δ̅ω̅ within time t is recorded as: $\overline{Δω} = \frac{{\sum }_{i = 1}^{n} {Δω}_{i}}{n} ,$ then: $θ \left(t\right) = {ω}_{0} ∗ t + \overline{Δω} ∗ t$

An angular deviation Δθ(t) between an actual rotation angle 0(t) and a theoretical rotation angle θ₀(t) = ω₀ * t at time t is: $Δθ \left(t\right) = θ \left(t\right) - {θ}_{0} \left(t\right) = \overline{Δω} ∗ t$

It can be concluded from the above expression that accuracy of the actual rotation angle is jointly determined by a speed fluctuation and the duration required for the rotor 50 to complete one revolution. Assuming that the speed fluctuation of the rotor 50 is controlled within a certain range, shortening the duration required for the rotor 50 to complete one revolution can ensure that the actual rotation angle deviation does not exceed a specified requirement. Similarly, for a revolution completed within a specified time, if the speed fluctuation is sufficiently small, it can also be ensured that the actual rotation angle deviation does not exceed the specified requirement.

Based on the above analysis, to reduce a deviation of the actual rotation angle feedback, one 360° revolution of the rotor 50 may be equally divided into several parts, so that an operating time for one rotation is divided into a sum of several parts of time. Due to the speed fluctuation of the rotor 50, the speed fluctuation depends on rigidity design of a mechanical transmission structure and adjustment capability of a motor speed. The present disclosure adopts a belt transmission structure. After dividing 360° into several equal angles, accuracy of an actual rotation angle fed back for each equal part is calculated by taking a speed fluctuation of 0.5% of the rotor 50 as an example.

Assuming that a number of images acquired per revolution by the CT device is 4800, dividing the 360° revolution into 100 segments means that a theoretical rotation angle per segment is 360°/100 = 3.6°, a number of samples required per segment is 4800/100 = 48, and a duration required for the rotor 50 to complete one revolution is 0.5 s. For each equal part, since accumulated error is maximum at a last field of view of each equal part, an angular deviation between an actual rotation angle and a theoretical rotation angle at the last field of view of each equal part is calculated. If the accumulated error at the last field of view of each equal part can meet a requirement, angular deviations at any field of view before the equal part can also meet the requirement. Angular deviation = theoretical rotation duration corresponding to last field of view × actual average angular speed - theoretical rotation angle, where: a theoretical rotation duration corresponding to the last field of view of each equal part is 0.5 s/100 = 5 ms, an actual average angular speed is 3.6°/(5 ms × (1+0.5%)) = 0.7164°/ms, and a theoretical rotation angle is 360°/4800 × 48 = 3.6°. It can be obtained that an angular deviation at the last field of view of each equal part is 5 ms × 3.6°/(5 ms × (1+0.5%)) - 3.6° = -0.0179° < 65 arcseconds. Such error is sufficient to meet an accuracy requirement of the CT device for rotation angle feedback, and reliability can reach 8020320 hours of continuous use.

Optionally, the power device 30 may include a motor 301 and a transmission belt 302.

In an embodiment of the present disclosure, the system for rotation angle feedback includes a detection module and a processing module. A detection module and a plurality of indexing marks arranged along a circumference centered on a scanning center of the CT device are disposed on a gantry of the CT device. The detection module is configured to detect the indexing marks during rotation of the rotor to generate a pulse signal, and transmit the pulse signal to the processing module. The processing module is configured to generate counting pulses corresponding to a present angular speed, and determine a target rotation angle corresponding to each acquired image during the rotation of the rotor based on the counting pulses between the indexing marks. According to the system for rotation angle feedback provided by the embodiment of the present disclosure, a pulse signal is obtained by disposing a plurality of indexing marks uniformly on the gantry and detecting each indexing mark by the detection module, so as to determine a target rotation angle based on pulse signals output by at least two of the indexing marks. Compared with a conventional rotation angle feedback technology, costs are greatly reduced and accuracy requirements of the CT device for rotation angle feedback are met.

FIG. 6 is a schematic diagram of a system for rotation angle feedback according to another embodiment. As shown in FIG. 6, the processing module 20 further includes an acquisition module 201 and a synchronization module 202. The acquisition module 201 is connected to the detection module 10 and a slip ring 2021, respectively. The synchronization module 202 is connected to the slip ring 2021 and a driving module 203, respectively. The acquisition module 201 is configured to generate counting pulses corresponding to a present angular speed. The acquisition module 201 may further transmit the pulse signal and the counting pulses to the synchronization module 202 via the slip ring 2021. The synchronization module 202 may synchronously transmit the pulse signal to the driving module 203. The acquisition module 201 or the synchronization module 202 may determine a count value corresponding to each acquired image based on the counting pulses between the indexing marks. The driving module 203 may control an angular speed of the rotor 50 based on the count value and a theoretical count value.

In an embodiment of the present disclosure, on a stator side of the CT device, a circle of blind holes 401 are uniformly formed on the stator 40 centered on the scanning center, and a position of each blind hole 401 corresponds to a target rotation angle within a 360° rotation range of the CT tube. A detection module 10 capable of detecting the blind holes 401 is mounted on a rotor side of the CT device, and angle information of the blind holes 401 is acquired using the detection module 10. When the rotor 50 starts to rotate, the detection module 10 starts to detect a pulse signal of each blind hole 401 on the stator 40. The acquisition module 201 transmits the detected pulse signal to a general-purpose processing unit (General Purpose Control, GPC) circuit (i.e., the synchronization module 202) on the stator side via a high-speed downlink (i.e., the slip ring 2021). The GPC circuit accesses the pulse signal to a main encoder interface circuit of a motor driver (i.e., the driving module 203), for speed regulation of the motor driver in a speed mode, so as to keep a speed fluctuation of the rotor 50 of the CT device within a small range and achieve an effect of fast synchronization of the pulse signal.

Optionally, the synchronization module 202 may also be a field programmable gate array (FPGA) hardware interface circuit.

With reference to FIG. 4, the detection module 10 transmits a pulse signal to the acquisition module 201. The acquisition module 201 acquires and latches a rising edge and a falling edge of the pulse signal in response to detecting the blind hole 401 to start counting, and then transmits the pulse signal and the generated counting pulses to the slip ring 2021. After detecting the rising edge and falling edge events, the slip ring 2021 transmits the events to the synchronization module 202 with a shortest time delay. The synchronization module 202 transmits the pulse signal to the driving module 203 to reduce transmission delays of the pulse signal. The synchronization module is configured to perform low-delay synchronization and level alignment on the pulse signal transmitted from the acquisition module via the slip ring. Specifically, the synchronization module receives the pulse signal including rising edge and falling edge events corresponding to the indexing marks, and synchronizes these edge events to an input terminal of a main encoder interface of the driving module in real time. By using hardware interface circuit synchronization, the synchronization module ensures that the pulse signal detected on the rotor side and the pulse signal received by the driving module on the stator side maintain high consistency in timing and level state, so as to minimize transmission delay and timing jitter. In this way, the driving module can obtain accurate and real-time rotation position feedback, so as to stably control the angular speed of the rotor and reduce rotation speed fluctuation.

In an exemplary embodiment, the count value is used to instruct the driving module 203 to control an angular speed of the rotor 50 based on the count value and a theoretical count value.

In an embodiment of the present disclosure, after receiving pulse signals of at least two of the indexing marks, the acquisition module 201 or the synchronization module 202 determines a count value based on the counting pulses. The driving module 203 adjusts an angular speed of the rotor 50 based on the count value and the theoretical count value, so as to reduce speed fluctuation of the motor and ensure uniform rotation of the rotor 50. The theoretical count value may be a total number of equal-time counting pulses that should be generated between two indexing marks in an ideal uniform speed state.

In a possible implementation, the acquisition module 201 may further generate counting pulses based on a rising edge or a falling edge of two adjacent blind holes. The acquisition module 201 or the synchronization module 202 determines a count value based on the counting pulses, and determines an actual rotation duration between the two blind holes based on the count value. The driving module 203 may adjust a present angular speed of the rotor based on a difference between an actual rotation duration and a theoretical rotation duration between the two blind holes.

In an embodiment of the present disclosure, the driving module may control an angular speed of the rotor based on the count value and the theoretical count value, so that a count value determined based on counting pulses of the present angular speed is more accurate.

In an embodiment, the acquisition module is configured to determine a counting duration required for each count value based on a first ratio of a duration required for one revolution of the rotor to a number of counting pulses per revolution of the rotor, and determine a number of minimum clock cycles required for generating the count value based on a second ratio of the counting duration required for each count value to a minimum clock cycle for the acquisition module.

Specifically, the acquisition module 201 is configured to use the second ratio as the number of minimum clock cycles required for generating the count value on a condition that the second ratio is an integer, and compensate the second ratio based on a remainder in the second ratio to obtain the number of minimum clock cycles required for generating the count value on a condition that the second ratio is a non-integer.

The minimum clock cycle of the acquisition module 201 is determined based on a frequency of the acquisition module. For example, on a condition that a crystal oscillator of the acquisition module is 125 MHz, the minimum clock cycle is 1/125 MHz = 8 ns. On a condition that the crystal oscillator of the acquisition module is 100 MHz, the minimum clock cycle is 1/100 MHz = 10 ns.

Optionally, the number of isochronous counting pulses per revolution may be 57600 or 62400.

Optionally, the duration required for the rotor to complete one revolution is determined based on parameters of a scanning protocol, and may be 0.5 s or 0.6 s.

In an embodiment of the present disclosure, assuming that the duration required for the rotor to complete one revolution is 0.5 s, i.e., 500000000 ns, the minimum clock cycle is 8 ns, and the number of isochronous counting pulses per revolution of the rotor is 57600, a counting duration required for the count value is 500000000/57600, which is a duration from one count value to a next count value. A number of minimum clock cycles required for generating the count value is determined based on a second ratio of the counting duration required for the count value to the minimum clock cycle, where the second ratio is 500000000 / (57600×8).

On a condition that the second ratio is an integer, the second ratio is used as the number of minimum clock cycles required for generating the count value. For example, in the embodiment corresponding to FIG. 3, the duration required for one revolution of the rotor is 499968000 ns, the number of isochronous counting pulses per revolution of the rotor is 57600, and the minimum clock cycle is 8 ns, so the second ratio is 499968000 / (57600×8) = 1085.

On a condition that the second ratio is a non-integer, the second ratio is compensated based on a remainder in the second ratio to obtain the number of minimum clock cycles required for generating the count value. For example, the above 500000000 / (57600×8) = 1085......32000 (remainder), and the second ratio is compensated using the following interpolation algorithm.

Specifically, the remainder is evenly distributed into 57600 equal-time counting pulses, i.e., 32000 / (57600×8) = 0.069444444 ns. The distributed remainder is accumulated from a first count value, and compensation is performed as long as the accumulated value is greater than 1. Since 0.0694×15 = 1.041 ns, one count value is supplemented every 15 count values to correct and compensate for accumulated errors of previous counting, so as to ensure accuracy of generating each count value and further ensure accuracy of angle feedback information under each field of view.

In an embodiment of the present disclosure, the acquisition module is configured to determine a counting duration required for each count value based on a first ratio of a duration required for one revolution of the rotor to a number of counting pulses per revolution of the rotor. The acquisition module is further configured to determine a number of minimum clock cycles required for generating the count value based on a second ratio of the counting duration required for each count value to a minimum clock cycle for the acquisition module, improving accuracy of determining the count value.

In an exemplary embodiment, a method for rotation angle feedback applied to a system for rotation angle feedback is provided. The system includes a processing module and a detection module, a plurality of indexing marks arranged along a circumference centered on a scanning center of a CT device and the detection module being disposed on a gantry of the CT device. The gantry includes a stator and a rotor, the method includes: in response to receiving the pulse signal corresponding to one of the indexing marks, determine a target rotation angle corresponding to an acquired image based on a present angular speed and a rotation duration from the one of the indexing marks, the pulse signal being generated and transmitted by the detection module in response to detecting the indexing marks during rotation of the rotor.

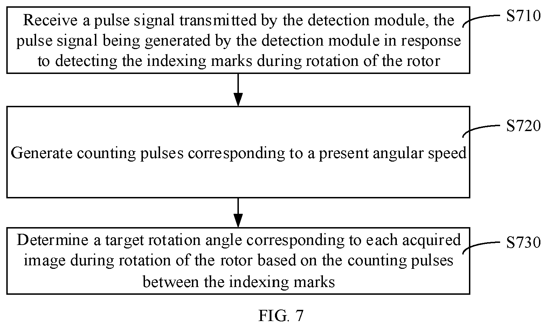

In an exemplary embodiment, as shown in FIG. 7, a method for rotation angle feedback is provided, which is described by taking an example applied to the system for rotation angle feedback in FIG. 3, and includes the following steps S710 to S730. The system includes a processing module 20 and a detection module 10, and a plurality of indexing marks arranged along a circumference centered on a scanning center of the CT device and the detection module are disposed on a gantry of the CT device. The gantry includes a stator and a rotor.

In step S710, a pulse signal transmitted by the detection module is received, the pulse signal being generated by the detection module in response to detecting the indexing marks during rotation of the rotor.

In step S720, counting pulses corresponding to a present angular speed are generated.

In step S730, a target rotation angle corresponding to each acquired image during the rotation of the rotor is determined based on the counting pulses between the indexing marks.

In the above method for rotation angle feedback, a pulse signal transmitted by a detection module is received, counting pulses corresponding to a present angular speed are generated in response to pulse signals output by the detection module when detecting at least two of the indexing marks, a count value corresponding to each acquired image is determined based on the counting pulses, and a target rotation angle corresponding to each acquired image during the rotation of the rotor is determined based on the count value and a counting duration required for each count value. In the embodiment of the present disclosure, a pulse signal is obtained by detecting each indexing mark using the detection module, so as to determine a target rotation angle based on pulse signals output by detecting at least two of the indexing marks. Compared with a conventional rotation angle feedback technology, costs are greatly reduced and accuracy requirements of the CT device for rotation angle feedback are met.

In some embodiments, the method further includes: determining a count value corresponding to each acquired image based on the counting pulses, and determining the target rotation angle corresponding to each acquired image during the rotation of the rotor based on each count value and a counting duration required for each count value.

In some embodiments, the method further includes: determining a counting duration required for generating each count value based on a first ratio of a duration required for one revolution of the rotor to a number of counting pulses per revolution of the rotor, and determining a number of minimum clock cycles required for generating the count value based on a second ratio of the counting duration required for generating each count value to a minimum clock cycle for the processing module.

In some embodiments, the method further includes: using the second ratio as the number of minimum clock cycles required for generating the count value on a condition that the second ratio is an integer, and compensating the second ratio based on a remainder in the second ratio to obtain the number of minimum clock cycles required for generating the count value on a condition that the second ratio is a non-integer.

In some embodiments, the method further includes feeding back the target rotation angle to a controller of the CT device.

In some embodiments, the at least two of the indexing marks include two adjacent indexing marks, and the method further includes generating the isochronous counting pulses between rising edges or falling edges of pulse signals corresponding to the two adjacent indexing marks.

It should be understood that although the steps in the flowchart of the above embodiments are sequentially displayed as indicated by arrows, the steps are not necessarily executed sequentially in the order indicated by the arrows. Unless explicitly specified herein, execution of the steps is not strictly limited in order, and the steps may be executed in other orders. Moreover, at least some of the steps in the embodiments described above may include a plurality of steps or stages. These steps or stages are not necessarily executed at the same time, but may be executed at different times. These steps or stages are not necessarily executed sequentially, but may be executed alternately or in turn with at least part of other steps or stages.

In some embodiments, a CT device is further provided in the present disclosure. The CT device includes a gantry, a plurality of indexing marks, a sensor, and a controller. The gantry includes a stator and a rotor. The plurality of indexing marks are provided on one of the stator and the rotor and are arranged at angular intervals along a circumference centered on a scanning center of the CT device. The sensor is provided on the other of the stator and the rotor and is configured to detect the indexing marks during rotation of the rotor to generate a pulse signal. The controller is configured to, in response to receiving the pulse signal corresponding to one of the indexing marks, determine a target rotation angle corresponding to an acquired image based on a present angular speed and a rotation duration from the one of the indexing marks.

In some embodiments, the controller is further configured to generate counting pulses corresponding to the present angular speed , and determine the target rotation angle corresponding to each acquired image during the rotation of the rotor based on the counting pulses between the indexing marks.

In some embodiments, the indexing marks include at least one of blind holes, raised structures, magnetic marks, and optical marks.

In some embodiments, the sensor includes a proximity switch. The proximity switch is configured to detect the indexing marks on the stator without contact during rotation of the rotor.

In some embodiments, the controller is further configured to determine a count value corresponding to each acquired image based on the counting pulses, and determine the target rotation angle corresponding to each acquired image during the rotation of the rotor based on each count value and a counting duration required for each count value.

In some embodiments, the CT device further includes a slip ring and a driving module. The controller includes an acquisition module connected to the sensor and the slip ring, respectively, and a synchronization module connected to the slip ring and the driving module.

In some embodiments, the acquisition module is configured to transmit the pulse signal and the counting pulses to the synchronization module through the slip ring. The synchronization module is configured to transmit the pulse signal to the driving module.

In some embodiments, the acquisition module is configured to determine, based on a first ratio of a duration required for one revolution of the rotor to a number of counting pulses per revolution of the rotor, a counting duration required for generating each count value, and determine, based on a second ratio of the counting duration required for generating each count value and a minimum clock cycle for the acquisition module, a number of minimum clock cycles required for generating the count value.

In some embodiments, the acquisition module is further configured to use the second ratio as the number of minimum clock cycles required for generating the count value on a condition that the second ratio is an integer, and compensate the second ratio based on a remainder in the second ratio to obtain the number of minimum clock cycles required for generating the count value on a condition that the second ratio is a non-integer.

In some embodiments, the plurality of indexing marks include a plurality of blind holes arranged at equal angular intervals around the scanning center.

In some embodiments, the plurality of blind holes include one zero-position hole and a plurality of positioning holes, and the sensor includes a first sensor configured to detect the zero-position hole and a second sensor configured to detect the plurality of positioning holes.

In some embodiments, the at least two of the indexing marks include two adjacent indexing marks, and the acquisition module is configured to generate the counting pulses between rising edges or falling edges of pulse signals corresponding to the two adjacent indexing marks.

In some embodiments, the synchronization module is further configured to determine an actual rotation duration between the adjacent indexing marks based on the count value, and transmit the actual rotation duration to the driving module. The driving module is configured to control the angular speed of the rotor based on a difference between the actual rotation duration and a theoretical rotation duration between the adjacent indexing marks.

In an exemplary embodiment, an electronic device including a memory and a processor is provided. The memory stores a computer program, and the processor implements steps of the method in any one of the above method embodiments when executing the computer program.

In an embodiment, a non-transitory computer-readable storage medium having a computer program stored thereon is provided. The computer program, when executed by a processor, causes the processor to implement steps of the method in any one of the above method embodiments.

In an embodiment, a computer program product including a computer program is provided. The computer program, when executed by a processor, causes the processor to implement steps of the method in any one of the above method embodiments.

It should be noted that user information (including but not limited to user device information, user personal information, etc.) and data (including but not limited to data for analysis, stored data, displayed data, etc.) involved in the present disclosure are information and data authorized by a user or fully authorized by all parties, and collection, use, and processing of relevant data need to comply with relevant laws, regulations, and standards.

A person of ordinary skill in the art may understand that all or part of processes in the methods of the above embodiments may be completed by instructing relevant hardware via a computer program. The computer program may be stored in a non-transitory computer-readable storage medium. When the computer program is executed, processes of the embodiments of the above methods may be included. Any reference to a memory, a database, or other media used in the embodiments provided in the present disclosure may include at least one of a non-transitory memory and a transitory memory. The non-transitory memory may include a read-only memory (ROM), a magnetic tape, a floppy disk, a flash memory, an optical memory, a high-density embedded non-transitory memory, a resistive random-access memory (ReRAM), a magnetoresistive random-access memory (MRAM), a ferroelectric random-access memory (FRAM), a phase change memory (PCM), a graphene memory, etc. The transitory memory may include a random-access memory (RAM) or an external cache. As an illustration and not a limitation, the RAM may be available in a variety of forms, such as a static random-access memory (SRAM) or a dynamic random-access memory (DRAM). The database involved in the embodiments provided in the present disclosure may include at least one of a relational database and a non-relational database. The non-relational database may include a blockchain-based distributed database, but is not limited thereto. The processor involved in the embodiments provided in the present disclosure may be a general-purpose processor, a central processing unit, a graphics processor, a digital signal processor, a programmable logic device, a quantum computing-based data processing logic device, an artificial intelligence (AI) processor, etc., but is not limited thereto.

## Claims

1. A system (100) for rotation angle feedback applied to a CT device, the system (100) comprising a detection module (10) and a processing module (20), the CT device having a gantry including a stator (40) and a rotor (50), the gantry being provided with the detection module (10) and a plurality of indexing marks arranged along a circumference centered on a scanning center of the CT device, wherein:
the detection module (10) is configured to detect the indexing marks during rotation of the rotor (50) to generate a pulse signal, and transmit the pulse signal to the processing module (20); and
the processing module (20) is configured to, in response to receiving the pulse signal corresponding to one of the indexing marks, determine a target rotation angle corresponding to an acquired image based on a present angular speed and a rotation duration from the one of the indexing marks;
optionally wherein the processing module (20) is further configured to generate counting pulses corresponding to the present angular speed, and determine the target rotation angle corresponding to each acquired image during the rotation of the rotor (50) based on the counting pulses between the indexing marks.

2. The system (100) according to claim 1, wherein the processing module (20) is further configured to determine a count value corresponding to each acquired image based on the counting pulses, and determine the target rotation angle corresponding to each acquired image during the rotation of the rotor (50) based on each count value and a counting duration required for each count value.

3. The system (100) according to claim 2, wherein the processing module (20) comprises:
an acquisition module (201) connected to the detection module (10) and a slip ring (2021) of the CT device, respectively; and
a synchronization module (202) connected to the slip ring (2021) and a driving module (203) of the CT device.

4. The system (100) according to claim 3, wherein the count value is used to instruct the driving module (203) to control the angular speed of the rotor (50) based on the count value and a theoretical count value.

5. The system (100) according to any one of claims 3 to 4, wherein the acquisition module (201) is configured to transmit the pulse signal and the counting pulses to the synchronization module (202) through the slip ring (2021); and
the synchronization module (202) is configured to transmit the pulse signal to the driving module (203).

6. The system (100) according to any one of claims 3 to 5, wherein the acquisition module (201) is configured to
determine, based on a first ratio of a duration required for one revolution of the rotor (50) to a number of counting pulses per revolution of the rotor (50), a counting duration required for generating each count value, and
determine, based on a second ratio of the counting duration required for generating each count value and a minimum clock cycle for the acquisition module (201), a number of minimum clock cycles required for generating the count value;
optionally wherein the acquisition module (201) is further configured to
use the second ratio as the number of minimum clock cycles required for generating the count value on a condition that the second ratio is an integer, and
compensate the second ratio based on a remainder in the second ratio to obtain the number of minimum clock cycles required for generating the count value on a condition that the second ratio is a non-integer.

7. The system (100) according to any one of claims 1 to 6, wherein the plurality of indexing marks are disposed on one of the stator (40) and the rotor (50), and the detection module (10) is disposed on the other of the stator (40) and the rotor (50).

8. The system (100) according to any one of claims 1 to 7, wherein the plurality of indexing marks comprise a plurality of blind holes (401) arranged at equal angular intervals around the scanning center;
optionally wherein the plurality of blind holes (401) comprise one zero-position hole (4011) and a plurality of positioning holes (4012), and the detection module (10) comprises a first detection module (101) configured to detect the zero-position hole (4011) and a second detection module (102) configured to detect the plurality of positioning holes (4012).

9. The system (100) according to any one of claims 3 to 6, wherein the synchronization module (202) is further configured to feed back the target rotation angle to a controller (200) of the CT device.

10. The system (100) according to any one of claims 3 to 6, wherein the at least two of the indexing marks comprise two adjacent indexing marks, and the acquisition module (201) is configured to generate the counting pulses based on rising edges or falling edges of pulse signals corresponding to the two adjacent indexing marks.

11. The system (100) according to claim 10, wherein the synchronization module (202) is further configured to determine an actual rotation duration between the adjacent indexing marks based on the count value, and transmit the actual rotation duration to the driving module (203); and the actual rotation duration is used to instruct the driving module (203) to control the angular speed of the rotor (50) based on a difference between the actual rotation duration and a theoretical rotation duration between the adjacent indexing marks.

12. A method for rotation angle feedback applied to a system (100) for rotation angle feedback, the system (100) including a processing module (20) and a detection module (10), a plurality of indexing marks arranged along a circumference centered on a scanning center of a CT device and the detection module (10) being disposed on a gantry of the CT device, the gantry including a stator (40) and a rotor (50), the method comprising:
in response to receiving a pulse signal corresponding to one of the indexing marks, determine a target rotation angle corresponding to an acquired image based on a present angular speed and a rotation duration from the one of the indexing marks, the pulse signal being generated and transmitted by the detection module (10) in response to detecting the indexing marks during rotation of the rotor (50).

13. The method according to claim 12, wherein in response to receiving the pulse signal corresponding to one of the indexing marks, determine the target rotation angle corresponding to the acquired image based on the present angular speed and the rotation duration from the one of the indexing marks comprises:
generating counting pulses corresponding to the present angular speed (S720); and
determining the target rotation angle corresponding to each acquired image during the rotation of the rotor (50) based on the counting pulses between the indexing marks (S7370).

14. The method according to claim 13, further comprising:
determining a count value corresponding to each acquired image based on the counting pulses; and
determining the target rotation angle corresponding to each acquired image during the rotation of the rotor (50) based on each count value and a counting duration required for each count value.

15. A non-transitory computer-readable storage medium having a computer program stored thereon, wherein the computer program, when executed by a processor, causes the processor to implement the method according to any one of claims 12 to 14.
